## Europäisches Patentamt
## European Patent Office
## Office européen des brevets

(19)

(11) Veröffentlichungsnummer: **0 061 487**
**B1**

(12)

# EUROPÄISCHE PATENTSCHRIFT

(45) Veröffentlichungstag der Patentschrift:
02.05.85

(21) Anmeldenummer: 81902805.1

(22) Anmeldetag: 07.10.81

(86) Internationale Anmeldenummer:
PCT/EP 81/00159

(87) Internationale Veröffentlichungsnummer:
WO 82/01130 (15.04.82 Gazette 82/10)

(51) Int. Cl.⁴: **A 61 K 35/78, C 07 G 17/00**

(54) Extract aus Uncaria tomentosa (WILLD.) DC. zur therapeutischen Verwendung.

(30) Priorität: 07.10.80 AT 4971/80

(43) Veröffentlichungstag der Anmeldung:
06.10.82 Patentblatt 82/40

(45) Bekanntmachung des Hinweises auf die Patenterteilung:
02.05.85 Patentblatt 85/18

(84) Benannte Vertragsstaaten:
AT CH DE FR GB LI NL SE

(56) Entgegenhaltungen:
DE - B - 1 184 897

Chemical Abstracts, Band 82, Nummer 21, 26. Mai 1975, Columbus, Ohio (US) S.R. HEMINGWAY et al.: "Alkaloids from S.American species of Uncaria (Rubiacease) " Siehe Seite 274, Zusammenfassung 135680k, J. Pharm. Pharmacol. 1974, 26. Supp. 113P

(73) Patentinhaber: KEPLINGER, Klaus, Müllerstrasse 30, A-6020 Innsbruck (AT)

(72) Erfinder: KEPLINGER, Klaus, Müllerstrasse 30, A-6020 Innsbruck (AT)

(74) Vertreter: Torggler, Paul, Dr. et al, Patentanwälte Dr. Paul Torggler DDr. Engelbert Hofinger Wilhelm-Greil-Strasse 16, A-6020 Innsbruck (AT)

**Beschreibung**

Die therapeutische Verwendung des Extraktes besteht dabei insbesondere in der Behinderung lebender Zellen, die einen Organismus gefährden können. Hiezu zählen vor allem Tumorzellen, die ihren im Organismus zugeteilten Aufgaben nicht mehr gerecht werden können. Zur Beeinflussung derartiger Tumorzellen werden unter anderem auch Zytostatika verwendet, die neben der gewünschten Wirkung auf die Tumorzellen auch unerwünschte Beeinflussungen auf gesunde Zellen ausüben, so dass trotz einer Vielzahl von bekannten, aus natürlichen Vorkommen oder synthetisch hergestellten Stoffen eine der vorrangigen Aufgaben der pharmazeutischen Industrie darin liegt, weitere Mittel zu entwickeln.

Eine bei der Voruntersuchung eventuell geeigneter Wirkstoffe allgemein übliche Testmethode prüft in vitro den Einbau von $^3$H-Thymidin (Thymindesoxyriboxid) in Ascites-Sarkomzellen von Ratten und Mäusen, wie sie von Weitzel et al. beschrieben wird (Hoppe – Seyler's Zeitschrift der physiologischen Chemie 348, 433, 1967). Thymidin ist ein Zellkernbestandteil und z. B. in den Ascites-Sarkomzellen in weit grösserem Ausmass enthalten als in Normalzellen. Werden transformierte Zellen mit Thymidin inkubiert, so wird dieses in die Zellen eingebaut, und die Wirksamkeit des zu untersuchenden Mittels anhand der Hemmung des Thymidineinbaus geprüft. Da die Einbaurate auch zellabhängig ist, wurden Standardpräparate geschaffen, die zum Vergleichen herangezogen werden können, beispielsweise ein Präparat aus 4-Amino-$^{10}$-N-methyl-pteroylglutaminsäure, das unter dem Handelsnamen Methotrexat® (Lederle Laboratories Division, American Cyanamid C., New York) erhältlich ist, und eine zufriedenstellende Hemmung des Thymidineinbaus bewirkt.

Wie bereits erwähnt, stellt die Untersuchung der Thymidineinbaurate nur einen der ersten Schritte bei der Beurteilung für die Applikation eines Wirkstoffes dar, dem eine Reihe weiterer Untersuchungen folgen müssen. Es hat sich leider gezeigt, dass eine Vielzahl von Wirkstoffen, die im Thymidineinbautest eine grosse Inhibierungsrate bewirken, aus anderen Gründen (Toxizität, Schädigung des Immunsystems des Organismus usw.) nicht zur gewünschten Beeinflussung der Zellen herangezogen werden können. Dies gilt beispielsweise, wie bekannt, für verschiedene Gerbstoffe, die im Thymidineinbautest zufriedenstellend abschneiden.

Die Erfindung schlägt nun zur Verwendung als Arzneimittel in physiologisch unbedenklicher Form einen weitgehend gerbstofffreien Extrakt aus Wurzelteilen der Uncaria tomentosa (WILLD.) DC. aus der Ordnung der Gentianales mit gelbbrauner oder dunkelroter Färbung des frischen Bastes, die beispielsweise im politischen Bezirk Chanchamayo, Peru, vorkommt, vor, der im $^3$H-Thymidineinbautest in Ascites-Sarkomzellen der Maus nach Weitzel et al. eine Einbauhemmung des $^3$H-Thymidins bewirkt, die zumindest halb so gross ist wie die Einbauhemmung durch Methotrexat®.

Die zur Herstellung des Wirkstoffes geeignete Uncaria tomentosa (WILLD.) DC. gehört in die Familie der Rubiaceen und diese in die Ordnung der Gentianales. Eine kurze systematische Übersicht mag die Verwandtschaftsverhältnisse veranschaulichen:

Ordnung Gentianales:
a) Loganiaceae mit Strychnos (Indolalkaloide)
b) Buddlejaceae
c) Rubiaceae
d) (Naucleaceae)

Familie Rubiaceae
Über die Systematik der Familie gibt es noch sehr divergierende Ansichten. Die Gattung Uncaria ist nach VERDCOURT B. 1958 der Unterfamilie Rubioideae zuzurechnen.

Gattung Uncaria
Die Gattung umfasst ca. 60 Arten, die im tropischen Amerika, Afrika und Asien vorkommen. Verbreitungsschwerpunkt ist der südostasiatische Raum und Indonesien bis Neu Guinea – Australien. Von den südamerikanischen Arten (Uncaria guianensis und Uncaria tomentosa) ist bisher wenig bekannt. Insbesondere wurden bisher keine exakten botanischen Untersuchungen über mögliche Unterarten sowie deren Inhaltsstoffe durchgeführt. Aus Chemical Abstracts, Band 82 (1975), Seite 274, Referat 135680k ist zu entnehmen, dass die südamerikanischen Arten bestimmte Alkaloide enthalten, wobei Uncaria tomentosa eine grössere Anzahl verschiedener Alkaloide enthält. Untersuchungen über die Inhaltsstoffe von Uncaria tomentosa (WILLD.) DC. sind jedoch in der Literatur bis jetzt nicht beschrieben. Es ist dabei anzunehmen, dass einerseits ein Teil der in der Literatur angeführten Alkaloide ebenfalls enthalten ist, andererseits aber durchaus wahrscheinlich, dass auch noch andere, zusätzliche oder gegebenenfalls auch noch nicht bekannte Alkaloide vorliegen. Als ein bekanntes Beispiel dafür darf in diesem Zusammenhang auf die unterschiedliche Wirkung der Indol-Alkaloide von altweltlichen und neuweltlichen Strychnos-Arten hingewiesen werden. Für einen Vergleich mit Uncaria tomentosa (WILLD.) DC. sind zweifellos auch die von anderen Naucleae bzw. Naucleaceae bekannt gewordenen Stoffe wichtig. In zweiter Linie werden auch bei Rubiceae mehr oder weniger allgemein verbreitete Stoffgruppen wichtig sein. Schliesslich sind die bei den verschiedenen Gentianales-Familien, insbesondere bei den Loganiaceae vorkommenden Stoffklassen, zu berücksichtigen.

Art Uncaria tomentosa (WILLD.) DC.
Wie nun festgestellt werden konnte, kommt die Art Uncaria tomentosa (WILLD.) DC. in mehreren Unterarten hauptsächlich in Zentralamerika und westlichem Südamerika von Guatemala bis Peru vor, die sich in der Färbung des frischen Bastes voneinander unterscheiden. Bisher wurden Unterarten mit gelbbrauner und mit dunkelroter Bastfärbung näher untersucht, die am Ostabhang der Anden im politischen Bezirk Chanchamayo, Peru, im Nebelwald der leicht gebirgigen Landschaft der Flüsse Rio Chanchamayo und Rio Perené in einer Seehöhe von 500–1200 m wachsen. Eine Uncaria tomentosa (WILLD.) DC. mit gelbbrauner Färbung des frischen Bastes ist im Herbarium des Institutes für Botanik der Universität Graz, Österreich (G.Z.U), enthalten. Die Untersuchung einer weiteren Unterart mit heller Bastfärbung ist noch im Gange.

Bei erfindungsgemässen Extrakten konnten dabei verschiedene Wirkungen festgestellt werden, die nicht ohne weiteres in einen erklärbaren Zusammenhang gebracht werden können:
1. Beeinflussung von Tumorzellen
2. kontrazeptive Wirkung
3. Entzündungshemmung

Ein Zusammenhang dieser unterschiedlichen Wirkungsweisen könnte dann gesehen werden, wenn Tumorzellen, Keimzellen und entzündete Zellen als undifferenzierte Zellen betrachtet werden, also als Zellen, die eine definierte, zugeordnete Aufgabe im Organismus noch nicht besitzen (Keimzellen), nicht mehr besitzen (Tumorzellen) oder vorübergehend nicht besitzen (entzündete Zellen). Die Gemeinsamkeit der Wirkungsweisen liegt demzufolge möglicherweise darin, dass die genannten undifferenzierten Zellen in ihrem Wachstum bzw. ihrer Teilung zumindest behindert werden.

Es ist zu vermuten, dass die erfindungsgemässen Mittel in der Lage sind, geschwächte Wasserstoffbindungen in der Desoxyridbonucleinsäure zwischen Purinen und Pyrimidinen zu festigen, und so über das rediculo-endotheliale System die mitose Teilung der Zellen zu inhibieren, bzw. die Produktion von Enzymen zu korrigieren, sowie die Reifeteilung von Keimzellen zu behindern.

Folgende Untersuchungen über die Wirksamkeit der erfindungsgemässen Extrakte wurden durchgeführt:

I. Die Toxizität
Oral liegt die mittlere lethale Toxizität für Mäuse bei einem Wert grösser als 16 g/kg Körpergewicht. Intraperetoneal sind 300 mg/kg noch unbedenklich, 1000 mg/kg führen nach 17–24 Stunden, 3000 mg/kg nach 4–16 Stunden zum Tod. Intravenös liegt die Toxizitätsgrenze bei 400 mg/kg Körpergewicht.

II. Die antiinflammatorische Wirkung
1. Die Wirkung wurde bei Mäusen an einem Carragenin-Pfotenödem ausgetestet. Als Vergleichssubstanz wurde Indometazin verwendet. Die Gabe der Testsubstanzen erfolgte eine Stunde vor der Gabe von Carragenin.

| Erfindungsgem. Präparat | Entzündungshemmung | Indometazin | Entzündungshemmung |
|---|---|---|---|
| i.p. 10 mg/kg | 47% | i.p. 3 mg/kg | 34% |
| i.p. 100 mg/kg | 49% | i.p. 9 mg/kg | 43% |
| p.o. 1 mg/kg | 0% | | |
| 2 mg/kg | 16% | | |
| 3 mg/kg | 33% | | |
| 10 mg/kg | 38% | | |
| 100 mg/kg | 42% | | |

In Dosierung von 200 mg täglich wurde über drei Wochen ein erfindungsgemässes Mittel in wässriger Lösung oral an zwei Patienten mit Gastritis verabreicht. Schon nach drei Tagen waren alle subjektiven Beschwerden beseitigt, die objektiven Befunde normalisierten sich im Behandlungszeitraum. Die gleiche Therapie führte bei einem Patienten mit Ulcus duodeni zum gleichen Ergebnis.

Im rheumatoiden Formenkreis sind zwei Fälle über kurze Zeit mit guten Erfolgen behandelt worden, doch konnte diese Arbeit nicht weiterverfolgt werden.

III. Orientierende Untersuchung über kontrazeptive Wirkung
Je fünf weibliche Mäuse bekamen p. o. täglich 25 mg/kg und 6,25 mg/kg erfindungsgemässes Präparat in wässriger Lösung. In einem Teil der Käfige wurden am 2. Tag eine männliche Maus eingesetzt, in den anderen am 9. Tag. In den Käfigen mit der Dosierung 25 mg/kg und 6,25 mg/kg gab es keine Jungen.

IV. Beeinflussung transformierter Zellen

1. Menschliche diploide Fibroblasten – Hela-Zellen
Menschliche diploide Fibroblasten von Hautbiopsien und etablierte Hela-Zellen wurden unter Standard-Kulturbedingungen (MEM, 10% fetal calf serum) gezogen. Zusatz von 0,4 g/ml erfindungsgemässem Extrakt zum Wuchsmedium hatte keine bzw. kaum morphologische Veränderungen an Fibroblasten zur Folge. Hela-Zellen rundeten sich nach dem Umsetzen ab, wurden blasig, lösten sich von der Platte ab. Dieser Vergleich menschlicher untransformierter Fibroblasten und Hela-Zellen wurde verschiedene Male mit wechselnden Konzentrationen von erfindungsgemässem Extrakt durchgeführt. Qualitativ war das Ergebnis immer wie oben beschrieben. Diese Experimente wurden auch fotografisch dokumentiert.

2. 3T3 – SV 3T3
Im ersten Experiment waren zwei menschliche Zellen verglichen worden, von denen eine Linie Fibroblasten waren, die andere Linie (Hela) aber aus einem Cervix-Carcinom stammte. Um möglichst nichttransformierte und transformierte Zellen gleichen Ursprungs zu vergleichen, wurden 3T3-Zellen und mit SV 40 transformierte 3T3-Zellen gezogen und parallel mit erfindungsgemässem Extrakt behandelt. Das Ergebnis war ähnlich wie im ersten Experiment-Typ. 3T3-Zellen werden wesentlich weniger geschädigt als SV 3T3-Zellen. Es ist jedoch bemerkenswert, dass 3T3-Zellen von erfindungsgemässen Extrakten stärker beeinflusst werden als primäre Fibroblastenkulturen. Die Ursache könnte darin liegen, dass 3T3 eine permanente Zellinie ist.

In allen Versuchsreihen, die zwischen Januar 1978 und Oktober 1978 durchgeführt wurden, zeigte sich deutlich, dass transformierte Zellen von erfindungsgemässen Extrakten mehr geschädigt wurden als nichttransformierte Zellen.

Im Zuge dieser oben beschriebenen Testreihen hat sich gezeigt, dass auch transformierte Zellen unter dem Einfluss von erfindungsgemässem Extrakt nicht ganz absterben, sondern ihre Mitoserate von etwa 70/1000 auf etwa 12/1000 sinkt. So behandelte transformierte Zellen wurden anschliessend auf einen harten Nährboden gesetzt, wo sie – wie nichttransformierte Zellen – abstarben, während unbehandelte transformierte Zellen auf einem harten Nährboden allgemein nicht absterben.

3. $^3$H-Thymidineinbautest in Ascitas-Sarkomzellen von Ratten nach Weitzel et al.

Es wurden folgende Thymidineinbautests durchgeführt, wofür

a) ein alkoholischer Gesamtextrakt aus Wurzeln einer ersten Pflanze der Unterart Uncaria tomentosa mit gelbbrauner Färbung des frischen Bastes,

b) ein Extrakt aus Wurzeln der ersten Pflanze der Unterart Uncaria tomentosa mit gelbbrauner Färbung des frischen Bastes, den durch eine basische Bleiacetatfällung die Hauptmenge an polymeren Catechingerbstoffen entzogen worden waren,

c) ein zweiter alkoholischer Gesamtextrakt aus Wurzeln einer zweiten Pflanze der Unterart Uncaria tomentosa mit gelbbrauner Färbung des frischen Bastes,

d) ein Äthylacetat-Macerat aus Wurzeln einer zweiten Pflanze der Unterart Uncaria tomentosa mit gelbbrauner Färbung des frischen Bastes,

e) ein alkoholischer Gesamtextrakt aus Wurzeln einer Pflanze der Unterart Uncaria tomentosa mit dunkelroter Färbung des frischen Bastes, und

f) ein Äthylacetat-Macerat aus Wurzeln einer Pflanze der Unterart Uncaria tomentosa mit dunkelroter Färbung des frischen Bastes verwendet wurden. Vergleichstests wurden mit dem erwähnten Methotrexat® durchgeführt.

Herstellung von Zellsuspensionen:

Die Transplantation von Walker-256-Carcinosarkom erfolgt in Abständen von 3–4 Tagen auf männliche oder weibliche ca. 200 g schwere Wistarratten durch Implantation von 0,2 ml Nativascites. Einer Ratte mit starker Ascitesbildung (Implantation 4 Tage zuvor) wurde, nachdem sie mit Chloroform betäubt worden war, der Ascites aus der nicht geöffneten Peritonealhöhle abpunktiert. Die Asciteszellensuspension wurde bei 4°C 10 min und 1000 upm zentrifugiert und der Niederschlag abdekantiert. Der Zellniederschlag wurde in Medium L-15 (Hersteller Fa. Boehringer, Mannheim) resuspendiert, unter den gleichen Bedingungen erneut zentrifugiert, der Überstand abdekantiert und mit Medium L-15 soweit verdünnt, dass die Zellsuspension $1,5\times10^6$ Zellen/ml enthielt. Die Bestimmung der Zellzahl erfolgte in einer Neubauer-'schen Zählkammer.

In vitro-Bestimmung der Hemmung der DNS-Synthese: Die Inkubation der Zellsuspension mit $^3$H-Thymidin erfolgte nach Weitzel und Mitarb. Ein Inkubationsansatz enthielt:

1 ml Zellsuspension des Walker-256-Carcionsarkom-Ascites ($1,5\times10$ Zellen), 1 mg Substanz in 0,5 ml phys. NaCl-Lsg. mit Ultraschall suspendiert und 0,1 ml $^3$H-Thymidin (spez. Akt. 50,8 Ci/m Mol) entsprechend 1 $\mu$ Ci.

Die Kontrolle enthielt 1,0 ml Zellsuspension, 0,5 ml Medium L-15 und 0,1 ml (1 $\mu$ Ci) radioaktiven Präkursor.

Pro Ansatz wurden vier bis sieben Proben inkubiert. Die Zellsuspension wurde zwei Stunden bei 37°C im Schüttelbad mit den in physiologischer Kochsalzlösung unter Ultraschall suspendierten Substanzen vorinkubiert. Anschliessend erfolgte die einstündige Inkubation mit dem Präkursor.

Nach dieser Zeit wurde der Einbau gestoppt, indem der Ansatz mit 1,0 ml eiskalter $NHClO_4$ versetzt wurde. Dadurch erfolgte die Lyse der Zellen und Fällung der säureunlöslichen Fraktion. Man liess den Ansatz über Nacht stehen. Die Niederschläge, die die eingebauten Präkursoren enthielten, wurden anschliessend durch Glasfaserfilter ($\varnothing$ = 25 mm) abgesaugt, zweimal mit eiskalter 1 $NHClO_4$-Lösung und einmal mit Äthanol gewaschen. Die Filter mit dem Niederschlag wurden in Polyäthylenfläschchen gebracht und die Aktivitäten im Toluol-Scintillator gemessen. Die Scintillationslösung enthielt pro Liter: 50 ml Liquifluor, 400 ml Triton X-100® und 550 ml Toluol p.a. $^3$H-Thymidin-Liquidfluor, Triton X-100® wurden von der Fa. NEN-Chemicals, Dreieichenhain, BRD, bezogen. Die Ergebnisse wurden gegen die Kontrolle verglichen und die Hemmeffekte in % ausgedrückt.

Das Ergebnis ist in nachstehender Tabelle zusammengefasst:

| getestete Substanz | Färbung des frischen Bastes | Inhibierung der $^3$H-Thymidin-Einbaurate (Mittelwert) |
|---|---|---|
| a (mit Gerbstoffen) | gelbbraun | 52,5% |
| b (weitgehend gerbstofffrei) | gelbbraun | 59,3% |
| Methotrexat® | | 45% |
| c (mit Gerbstoffen) | gelbbraun | 81,2% |
| d (weitgehend gerbstofffrei) | gelbbraun | 80,1% |
| Methotrexat® | | 73,3% |
| e (mit Gerbstoffen) | dunkelrot | 47% |
| f (weitgehend gerbstofffrei) | dunkelrot | 64,7% |
| Methotrexat® | | 73,3% |

Aus vorstehender Tabelle ergibt sich, dass die Einbauhemmung der Extrakte aus Uncaria tomentosa (WILLD.) DC. mit gelbbrauner Bastfärbung jeweils der von Methotrexat überlegen waren und über der von Extrakten aus Uncaria tomentosa (WILLD.) DC. mit dunkelroter Bastfärbung liegt. Letzteres beruht darauf, dass Uncaria tomentosa mit dunkelroter Bastfärbung den Wirkstoff in geringerer Konzentration enthält. Weiter ist daraus ersichtlich, dass die Wirkung der erfindungsgemässen Extrakte nicht auf den Gerbstoffen beruht, die, wie erwähnt, ebenfalls eine zufriedenstellende Einbauhemmung bewirken, sondern dass der Wirkstoff in dem zumindest weitgehend gerbstofffreien Extrakt enthalten ist.

4. Eine Reihe von Patienten wurden in der Dosierung von 200 mg täglich oral in wässriger Lösung mit einem erfindungsgemässen Extrakt über verschieden lange Zeiträume behandelt, wobei die normale Therapie (Bestrahlungen, Zytostatika) unverändert weitergeführt wurde. Es handelt sich um folgende Erkrankungen jeweils in einem sehr weit fortgeschrittenen Stadium: Melanom, Osteosarcom Collumcarcinom, Mammacarcinom, Hodgkin, Erythroleukämie. In allen Fällen hat sich gezeigt, dass während der Behandlung mit erfindungsgemässem Extrakt Zytostatica wesentlich besser vertragen wurden. Teilweise konnte mit der Dosierung in die Höhe gegangen werden, immer waren die Erholungsphasen um wesentliches kürzer. Bei der Erythroleukämie konnte auf Bluttransfusionen über lange Zeiträume überhaupt verzichtet werden.

Einige Werte des Melanom-Patienten zur Verdeutlichung:

Quantitative Immuglobulinbestimmung

| vor der Behandlung mit erfindungsgemässem Präparat | nach 8 Monaten |
| --- | --- |
| IgG 1.280 mg% | 2000 mg% |
| IgA 82 mg% | 174 mg% |
| IgN 186 mg% | 288 mg% |

Durchschnittliche Blutbefunde

| vor der Behandlung mit erfindungsgemässem Präparat | während der Behandlung mit erfindungsgemässem Präparat |
| --- | --- |
| Leukozyten 3.060 | 4.350 |
| Thrombozyten 90.800 | 92.300 |
| Hb 12,7 | 13,5 |

5. Wirkung bei gesunden Testpersonen

Die Testgruppe bestand aus 7 Personen (6 männlich, 1 weiblich). Verabreicht wurde ein erfindungsgemässer Extrakt in wässriger Lösung oral, 200 mg täglich am Morgen vor dem Frühstück. Als Parameter wurden herangezogen: ICT, Ery, Leuko, Thr. Hb, Hk, Sgot, Sgpt, Gamma-Gt, Cholinesterase, Alk. Phosphatase mU/ml Bili mg%, Neutralfett mg%, Harnsäure, Kreatinin, Harnstoff, Kalium, Ca, Na, Chloride, GE.

Am subjektiven Befinden der Testpersonen hat sich während des Behandlungszeitraumes konkret nichts geändert. Einige Parameter zeigten aber doch eine bemerkenswerte Veränderung:

| | | vor dem Test | am Ende des Tests |
| --- | --- | --- | --- |
| SGOT | 1 | 45 | 11 |
| | 2 | 19 | 6 |
| | 3 | 21 | 17 |
| | 4 | 8 | 11 |
| | 5 | kein Wert | 18 |
| | 6 | 6 | 13 |
| | 7 | 11 | 19 |
| SGPT | 1 | 98 | 7 |
| | 2 | 33 | 6 |
| | 3 | 49 | 3 |
| | 4 | 21 | 11 |
| | 5 | kein Wert | 5 |
| | 6 | 14 | 12 |
| SGPT | 7 | 23 | 11 |
| G-GT | 1 | 81 | 10 |
| | 2 | 66 | 30 |
| | 3 | 72 | 18 |
| | 4 | 14 | 17 |
| | 5 | kein Wert | 45 |
| | 6 | 11 | 4 |
| | 7 | 62 | 52 |
| Alka- | 1 | 197 | 22 |
| lische | 2 | 120 | 22 |
| Phos- | 3 | 98 | 29 |
| phate | 4 | 76 | 38 |
| | 5 | kein Wert | kein Wert |
| | 6 | 131 | 9 |
| | 7 | 87 | 16 |
| Harn- | 1 | 7,5 | 6,5 |
| säure | 2 | 8,2 | 7,7 |
| | 3 | 6,9 | 5,8 |
| | 4 | 6,4 | 5,0 |
| | 5 | 5,3 | kein Wert |
| | 6 | 4,3 | 3,8 |
| | 7 | 7,0 | 6,5 |

V. Inhaltsstoffe von Uncaria Arten

Inhaltsstoffe der Uncaria tomentosa (WILLD.) DC.

Verschiedene Extraktionsverfahren aus gemahlener Wurzel und Wurzelrinde einer Uncaria tomentosa (WILLD.) DC. mit gelbbrauner Färbung des frischen Bastes ergaben nach Entfernung des Extrahens:

Wasserextrakt ... 7,7 Gew.-% Trockensubstanz (braunes, lackartiges Produkt)

Äthanolextrakt ... 8,5 Gew.-% (wie oben)

Chloroformextrakt ... 1,0 Gew.-% (gelbe Schmiere)

Die nachstehenden Untersuchungen wurden grossteils mit Trockensubstanz aus wässrigem oder alkoholischem Extrakt durchgeführt.

a) Gerbstoffe: Ausfällung durch Bleiacetat, Verfärbung durch Eisen-III-Chlorid. Bei einer orientierenden DC-Untersuchung, bei der als Fliessmittel eine Mischung von 45% Toluol, 45% Aceton und 10% Ameisensäure eingesetzt wurde, kam es zu einer Auftrennung mehrerer Substanzen, jedoch war eine Identifizierung nicht möglich. Da eine Säurebehandlung (HCl) des Wasserextraktes eine Fällung hervorruft, dürfte es sich hier um Catechingerbstoffe, also um hydrolisierbare Gerbstoffe, handeln. Weitere Untersuchungen der Gerbstoffe wurden noch nicht vorgenommen (DC = Dünnschichtchromatographie).

b) Alkaloide: Konnten eindeutig nachgewiesen werden, sowohl chemisch als auch mit Hilfe der DC. Es handelt sich um mehrere Alkaloide, die im UV-256 absorbieren. Ein Alkaloid zeigte im UV-366 blaue Fluoreszenz. Eine Identifizierung der Alkaloide war noch nicht möglich.

c) Saponine: Der Hämolyseversuch verlief negativ. Ein Vorkommen von Saponinen kann jedoch nicht ausgeschlossen werden, weil ja bekanntlich Gerbstoffe die oben angeführte Bestimmung stören.

d) Unbekannte fluoreszierende Substanzen: Sowohl im Wasserextrakt als auch im Alkoholextrakt konnten mehrere grün und blau fluoreszierende Verbindungen (UV-366) gefunden werden. Substanzen mit ähnlichem, dünnschichtchromatographischem Verhalten treten auch nach der Hydrolyse des Wasserextraktes mit Salzsäure auf. Beim Behandeln mit Ammoniakdampf wurde bei diesen Substanzen keine Farbänderung beobachtet (Flavonoide daher unwahrscheinlich). Es ist möglich, dass es sich um Phenylpropanderivate handelt oder um Phenolsäuren. Eine dieser Substanzen konnte isoliert werden. Sie zeigt grüne Fluoreszenz (UV-366), hat einen Schmelzpunkt von 138°C (±2°) und dürfte etwa in der Grössenordnung von 0,18 Gew.-% (bezogen auf 1 kg Wurzel) vorhanden sein.

e) Kohlenhydrate: Im Wasserextrakt wurden geringe Mengen eines Zuckers (wahrscheinlich Glukose) gefunden. Nach der Hydrolyse mit Salzsäure konnten im Hydrolysat grössere Mengen dieses Zuckers dünnschichtchromatographisch nachgewiesen werden.

f) Weitere Substanzen: Nennenswerte Mengen von Stärke, Fetten, fetten Ölen, ätherischen Ölen und Anthrachinonderivaten können nach mikrochemischen Untersuchungen nicht ausgeschlossen werden.

Wie sich aus der Tabelle auf Seite 9 ergibt, sind weitgehend gerbstofffreie Extrakte in der Einbauhemmung den Gesamtextrakten zumindest gleichwertig, in zwei Beispielen (b und f) sogar wesentlich überlegen. Zur zumindest weitgehenden Entfernung der Gerbstoffe, die aufgrund ihrer Nebenwirkungen die Dosierung des Wirkstoffes bei der Applikation von Gesamtextrakten beschränken, wird beispielsweise vorgeschlagen, dass die Teile der Uncaria tomentosa alkalisiert und anschliessend mit einem organischen Lösungsmittel extrahiert werden. Im einzelnen wird dabei bevorzugt die fein gepulverte Droge mit 10% NH$_3$-Lösung angefeuchtet und bei Raumtemperatur ca. 1 Stunde stehengelassen. Nach Zugabe von Äthylacetat (etwa 2 bis 3-fache Menge des Gewichtes der eingesetzten Droge) wird in einem Erlenmeyer-Kolben eine halbe Stunde am Sorvallmixer extrahiert und anschliessend dekantiert. Der Drogenrückstand wird mit Äthylacetat gewaschen und nochmals der gleichen Behandlung unterzogen.

Anschliessend bleibt der Rückstand noch eine Woche in Äthylacetat-Lösung bei 12°C stehen. Das Filtrat ist noch schwach alkaloidhaltig. Es zeigt das gleiche Alkaloidmuster wie die Sorvallextraktionen. Die drei so erhaltenen Filtrate werden vereinigt. Zur Anreicherung des Wirkstoffes werden die Filtrate im Vakuum zu einem zähflüssigen braunen Konzentrat eingeengt. Die Ausbeute an Rohextrakten beträgt etwa 6 Gew.-% der eingesetzten Drogenmenge.

Nach einem weiteren erfindungsgemässen Vorschlag wird zur zumindest weitgehenden Entfernung der Gerbstoffe ein für den Organismus unbedenklicher Spaltpilz eingesetzt, mit dessen Hilfe bereits die Teile der Uncaria tomentosa selbst oder deren wässeriger Extrakt einer fermentativen Umwandlung unterzogen wird, die zumindest den grössten Teil der enthaltenen Gerbstoffe im ersten Fall unlösbar macht und im zweiten Fall die Gerbstoffe aus dem Extrakt ausfällt. Vorzugsweise handelt es sich bei dem Spaltpilz um einen Vertreter der Gattung Trichoderma, der der Gruppe der imperfekten Ascomyceten angehört und alle Eigenschaften eines Schimmelpilzes aufweist. Er ist aerob, türkis-blau und hat eine im wesentlichen flächige Ausbreitung. Das Wachstum des Spaltpilzes ist gut, unter den klimatischen Bedingungen Mittel- bzw. Südamerikas sind die ausgegrabenen Wurzeln der Uncaria tomentosa (WILLD.) DC. in frühestens 2 Tagen durchschimmelt. Bei Wirkstoffen, die aus mit vom Spaltpilz besiedelten Wurzeln der Uncaria tomentosa gewonnen wurden, konnten dünnschichtchromatographisch einerseits nur mehr geringe Spuren von Gerbstoffen und andererseits im UV-366 eine neue grün fluoreszierende Substanz festgestellt werden, die anstelle einer der oben unter V. d. beschriebenen, blau fluoreszierenden Substanzen getreten war.

Die fermentative Umwandlung erfolgt vorzugsweise in saurem Milieu, etwa bei pH 3, der z. B. durch Zugabe von verdünnter Zitronensäure erreicht werden kann.

Ein weiterer erfindungsgemässer Vorschlag zur zumindest weitgehenden Entfernung der Gerbstoffe besteht darin, dass die Teile der Uncaria tomentosa bei einer Temperatur, die knapp unter der Verkohlungstemperatur des Holzes liegt, vollständig getrocknet und anschliessend flüssig extrahiert werden. Dabei gehen die Gerbstoffe nicht mehr in Lösung.

Vorzugsweise erfolgt die Trocknung bei 130–150°C während eines Zeitraumes von etwa 45 min.

Der den Wirkstoff enthaltende Extrakt wird gegebenenfalls nach Anreicherung durch Konzentration oder Gefriertrocknung und Wiederlösung in einer physiologisch verträglichen Flüssigkeit, wie Wasser, zu gut verträglichen Arzneimitteln verarbeitet, die eine ausgezeichnete zytostatische, kontrazeptive und antiinflammatorische Wirkung aufweisen.

**Patentansprüche für die Vertragsstaaten:**
**CH, DE, FR, GB, LI, NL, SE**

1. Weitgehend gerbstofffreier Extrakt aus Wurzelteilen der Uncaria tomentosa (WILLD.) DC. aus der Ordnung der Gentianales, mit gelbbrauner oder dunkelroter Färbung des frischen Bastes, die beispielsweise im politischen Bezirk Chanchamayo, Peru, vorkommt, der im $^3$-H-Thymidineinbautest in Ascites-Sarkomzellen der Maus nach Weitzel et al. eine Einbauhemmung des $^3$-H-Thymidins bewirkt, die zumindest halb so gross ist wie die Einbauhemmung durch Methotrexat®, in physiologisch unbedenklicher Form zur Verwendung als Arzneimittel.

2. Extrakt nach Anspruch 1, dadurch gekennzeichnet, dass zumindest die resorbierbaren Gerbstoffe so weit reduziert sind, dass einerseits ihre Aktivität physiologisch unbedenklich ist, andererseits sie jedoch noch eine allfällige Funktion als Träger- und/oder Schutzsubstanz erfüllen können.

3. Extrakt nach Anspruch 1, dadurch erhältlich, dass zur weitgehenden Entfernung der Gerbstoffe die Wurzelteile der Uncaria tomentosa (WILLD.) DC. alkalisiert und anschliessend mit einem organischen Lösungsmittel, beispielweise Äthylacetat, extrahiert werden.

4. Extrakt nach Anspruch 1, dadurch erhältlich, dass zur weitgehenden Entfernung der Gerbstoffe die Wurzelteile der Uncaria tomentosa (WILLD.) DC. alkoholisch extrahiert werden, worauf der Extrakt mit Bleiacetatlösung versetzt und der entstehende Niederschlag entfernt wird.

5. Extrakt nach Anspruch 1, dadurch erhältlich, dass die Teile der Uncaria tomentosa (WILLD.) DC. wässrig extrahiert und der Extrakt zur weitgehenden Entfernung der Gerbstoffe mit Hilfe eines Spaltpilzes einer fermentativen Umwandlung unterzogen werden, worauf der entstehende Niederschlag entfernt wird.

6. Extrakt nach Anspruch 1, dadurch erhältlich, dass die Wurzelteile einer Uncaria tomentosa (WILLD.) DC. zur weitgehenden Entfernung der Gerbstoffe mit Hilfe eines Spaltpilzes einer fermentativen Umwandlung unterzogen und anschliessend flüssig extrahiert werden, worauf der den Wirkstoff enthaltene Extrakt in eine physiologisch unbedenkliche Form gebracht wird.

7. Extrakt nach Anspruch 5 oder 6, dadurch erhältlich, dass die fermentative Umwandlung in saurem Milieu, vorzugsweise bei einem pH-Wert zwischen 2 und 5, erfolgt.

8. Extrakt nach Anspruch 7, dadurch erhältlich, dass die fermentative Umwandlung in Anwesenheit von verdünnter Zitronensäure vorgenommen wird.

9. Extrakt nach einem der Ansprüche 5–8, dadurch erhältlich, dass die fermentative Umwandlung mit einem Vertreter der Gattung Trichoderma aus der Gruppe der imperfekten Ascomyceten vorgenommen wird.

10. Extrakt nach Anspruch 1, dadurch erhältlich, dass Wurzelteile einer Uncaria tomentosa (WILLD.) DC., zur weitgehenden Entfernung der Gerbstoffe bei einer Temperatur, die knapp unter der Verkohlungstemperatur des Holzes liegt, getrocknet und anschliessend flüssig extrahiert werden, worauf der den Wirkstoff enthaltende Extrakt in eine physiologisch unbedenkliche Form gebracht wird.

11. Extrakt nach Anspruch 10, dadurch erhältlich, dass die Trocknung bei 130–150°C während eines Zeitraumes von etwa 45 min vorgenommen wird.

12. Extrakt nach einem der Ansprüche 1 bis 10, dadurch erhältlich, dass er, nachdem er von den Gerbstoffen weitgehend befreit worden ist, konzentriert, vorzugsweise eingetrocknet und vorzugsweise im Extrahens wieder gelöst wird.

13. Extrakt nach einem der Ansprüche 1 bis 12, dadurch erhältlich, dass er gefriergetrocknet und die Trockensubstanz in einer physiologischen Flüssigkeit, beispielsweise Wasser, gelöst wird.

**Patentansprüche für den Vertragsstaat AT**

1. Verfahren zur Herstellung einer neuen, pharmazeutisch wirksamen Substanz, die im $^3$H-Thymidineinbautest in Ascites-Sarkomzellen der Maus nach Weitzel et al. eine Einbauhemmung des $^3$H-Thymidins bewirkt, die zumindest halb so gross ist wie die Einbauhemmung durch Methotrexat®, dadurch gekennzeichnet, dass Wurzelteile der Uncaria tomentosa (WILLD.) DC. aus der Ordnung der Gentianales, mit gelbbrauner oder dunkelroter Färbung des frischen Bastes, die beispielsweise im politischen Bezirk Chanchamayo, Peru vorkommt, flüssig extrahiert und von den Gerbstoffen weitgehend befreit werden, worauf der Extrakt in eine physiologisch unbedenkliche Form überführt wird.

2. Verfahren nach Anspruch 1, dadurch gekennzeichnet, dass zumindest die resorbierbaren Gerbstoffe so weit reduziert werden, dass einerseits ihre Aktivität physiologisch unbedenklich ist, andererseits sie jedoch noch eine allfällige Funktion als Träger- und/oder Schutzsubstanz erfüllen können.

3. Verfahren nach Anspruch 1, dadurch gekennzeichnet, dass zur weitgehenden Entfernung der Gerbstoffe die Wurzelteile der Uncaria tomentosa (WILLD.) DC. alkalisiert und anschliessend mit einem organischen Lösungsmittel, beispielsweise Äthylacetat, extrahiert werden.

4. Verfahren nach Anspruch 1, dadurch gekennzeichnet, dass zur weitgehenden Entfernung der Gerbstoffe die Wurzelteile der Uncaria tomentosa (WILLD.) DC. alkoholisch extrahiert werden, worauf der Extrakt mit Bleiacetatlösung versetzt und der entstehende Niederschlag entfernt wird.

5. Verfahren nach Anspruch 1, dadurch gekennzeichnet, dass die Teile der Uncaria tomentosa (WILLD.) DC. zur weitgehenden Entfernung der Gerbstoffe mit Hilfe eines Spaltpilzes einer fermentativen Umwandlung unterzogen wird, worauf der entstehende Niederschlag entfernt wird.

6. Verfahren nach Anspruch 1, dadurch gekennzeichnet, dass die Wurzelteile einer Uncaria tomentosa (WILLD.) DC. zur weitgehenden Entfernung der Gerbstoffe mit Hilfe eines Spaltpilzes einer fermentativen Umwandlung unterzogen und anschliessend flüssig extrahiert werden, worauf der den Wirkstoff enthaltende Extrakt in eine physiologisch unbedenkliche Form gebracht wird.

7. Verfahren nach Anspruch 5 oder 6, dadurch gekennzeichnet, dass die fermentative Umwandlung in saurem Milieu, vorzugsweise bei einem pH-Wert zwischen 2 und 5, erfolgt.

8. Verfahren nach Anspruch 7, dadurch gekennzeichnet, dass die fermentative Umwandlung in Anwesenheit von verdünnter Zitronensäure vorgenommen wird.

9. Verfahren nach einem der Ansprüche 5–8, dadurch gekennzeichnet, dass die fermentative Umwandlung mit einem Vertreter der Gattung Trichoderma aus der Gruppe der imperfekten Ascomyceten vorgenommen wird.

10. Verfahren nach Anspruch 1, dadurch gekennzeichnet, dass Wurzelteile einer Uncaria tomentosa (WILLD.) DC., zur weitgehenden Entfernung der Gerbstoffe bei einer Temperatur, die knapp unter der Verkohlungstemperatur des Holzes liegt, getrocknet und anschliessend flüssig extrahiert werden, worauf der den Wirkstoff enthaltende Extrakt in eine physiologisch unbedenkliche Form gebracht wird.

11. Verfahren nach Anspruch 10, dadurch gekennzeichnet, dass die Trocknung bei 130–150°C während eines Zeitraumes von etwa 45 min vorgenommen wird.

12. Verfahren nach einem der Ansprüche 1 bis 10, dadurch gekennzeichnet, dass der Extrakt, nachdem er von den Gerbstoffen weitgehend befreit worden ist, konzentriert, vorzugsweise eingetrocknet und vorzugsweise im Extrahens wieder gelöst wird.

13. Verfahren nach einem der Ansprüche 1 bis 12, dadurch gekennzeichnet, dass der Extrakt gefriergetrocknet und die Trockensubstanz in einer physiologischen Flüssigkeit, beispielsweise Wasser, gelöst wird.

**Patent claims for the contracting states:**
**CH, DE, FR, GB, LI, NL, SE**

1. Extract largely free of tanning substances from root parts of the Uncaria tomentosa (WILLD.) DC. of the order of the Gentianales with yellow brown or dark red colour of the fresh bast and found, for example, in the political district of Chanchamayo, Peru, which effects an incorporating inhibition of the $^3$H-thymidine in ascites-sarcoma cells of the mouse in the $^3$H-thymidine incorporation test according to Weitzel et al., which is at least half the incorporating inhibition through Methotrexat®, in physiologically acceptable form for use as a medicament.

2. Extract according to claim 1, characterized in that at least the resorbable tanning substances are reduced such that, on the one hand, their activity is physiologically acceptable but, that on the other hand, they are able to fulfil, if required, a function as a carrier and/or protective substance.

3. Extract according to claim 1, obtainable by alkalizing the root parts of the Uncaria tomentosa (WILLD.) DC. to remove the tanning substances to a great extent, and by subsequently extracting them with an organic solvent, for example ethyl acetate.

4. Extract according to claim 1, obtainable by extracting by alcohol the root parts of the Uncaria tomentosa (WILLD.) DC. to remove the tanning substances to a great extent, whereupon the extract is mixed with lead acetate solution and the resulting precipitate is removed.

5. Extract according to claim 1, obtainable by extracting by water the parts of the Uncaria tomentosa (WILLD.) DC. and by subjecting the extract to a fermentative transformation to remove the tanning substances to a great extent by means of schizomycetes, whereupon the precipitate is removed.

6. Extract according to claim 1, obtainable by subjecting the root parts of an Uncaria tomentosa (WILLD.) DC. to a fermentative transformation to remove the tanning substances to a great extent by means of schizomycetes and by subsequently extracting them by liquid, whereupon the extract containing the active substance is put into a physiologically acceptable form.

7. Extract according to claim 5 or 6, obtainable by effecting the fermentative transformation in acid medium, preferably at a pH-value between 2 and 5.

8. Extract according to claim 7, obtainable by effecting the fermentative transformation in the presence of diluted citric acid.

9. Extract according to one of claims 5–8, obtainable by effecting the fermentative transformation with a representative of the genus Trichoderma of the group of the imperfect Ascomycetes.

10. Extract according to claim 1, obtainable by drying root parts of an Uncaria tomentosa (WILLD.) DC. to remove the tanning substances to a great extent, at a temperature lying slightly below the carbonizing temperature of wood, and by subsequently extracting them by liquid, whereupon the extract containing the active substances is put into a physiologically acceptable form.

11. Extract according to claim 10, obtainable by effecting drying at 130–150°C over a period of about 45 min.

12. Extract according to one of claims 1 to 10, obtainable, after it has been freed from the tanning substances to a great extent, by concentrating, preferably by drying, and dissolving it again preferably in the extraction agent.

13. Extract according to one of claims 1 to 12, obtainable by freeze-drying and dissolving the dry substance in a physiological liquid, for example water.

**Claims for the contracting state: Austria**

1. Method for the production of a new, pharmaceutically active substance which effects an incorporating inhibition of the $^3$H-thymidine in ascites-sarcoma cells of the mouse in the $^3$H-thymidine incorporation test according to Weitzel et al., which is at least half the incorporating inhibition through Methotrexat®, characterized in that root parts of the Uncaria tomentosa (WILLD.) DC. of the order of the Gentianales with yellow brown or dark red colour of the fresh bast and found, for example, in the political district of Chanchamayo, Peru, are extracted by liquid and largely freed from the tanning substances, whereupon the extract is put into a physiologically acceptable form.

2. Method according to claim 1, characterized in that at least the resorbable tanning substances are reduced such that, on the one hand, their activity is physiologically acceptable but, that on the other hand, they are able to fulfil, if required, a function as a carrier and/or protective substance.

3. Method according to claim 1, characterized by alkalizing the root parts of the Uncaria tomentosa (WILLD.) DC., to remove the tanning substances to a great extent, and by subsequently extracting them with an organic solvent, for example ethyl acetate.

4. Method according to claim 1, characterized by extracting by alcohol the root parts of the Uncaria tomentosa (WILLD.) DC. to remove the tanning substances to a great extent, whereupon the extract is mixed with lead acetate solution and the resulting precipitate is removed.

5. Method according to claim 1, characterized by extracting by water the parts of the Uncaria tomentosa (WILLD.) DC. and by subjecting the extract to a fermentative transformation to remove the tanning substances to a great extent by means of schizomycetes, whereupon the precipitate is removed.

6. Method according to claim 1, characterized by subjecting the root parts of an Uncaria tomentosa (WILLD.) DC. to a fermentative transformation to remove the tanning substances to a great extent by means of schizomycetes and by subsequently extracting them by liquid, whereupon the extract containing the active substance is put into a physiologically acceptable form.

7. Method according to claim 5 or 6, characterized by effecting the fermentative transformation in acid medium, preferably at a pH-value between 2 and 5.

8. Method according to claim 7, characterized by effecting the fermentative transformation in the presence of diluted citric acid.

9. Method according to one of claims 5–8, characterized by effecting the fermentative transformation with a representative of the genus Trichoderma of the group of the imperfect Ascomycetes.

10. Method according to claim 1, characterized by drying root parts of an Uncaria tomentosa (WILLD.) DC. to remove the tanning substances to a great extent, at a temperature lying slightly below the carbonizing temperature of wood, and by subsequently extracting them by liquid, whereupon the extract containing the active substances is put into a physiologically acceptable form.

11. Method according to claim 10, characterized by effecting drying at 130–150°C over a period of about 45 min.

12. Method according to one of claims 1 to 10, characterized in that the extract is, after it has been freed from the tanning substances to a great extent, concentrated, preferably by drying and dissolved again preferably in the extraction agent.

13. Method according to one of claims 1 to 12, characterized by freeze-drying and dissolving the dry substance in a physiological liquid, for example water.

**Revendications pour les Etats contractants:
CH, DE, FR, GB, LI, NL, SE**

1. Extrait largement dépourvu de tanin provenant de parties de racines d'Uncaria tomentosa (WILLD.) DC. de l'ordre des gentianacées, avec coloration jaune-brun ou rouge foncé de la fibre fraîche, que l'on trouve par exemple dans le district de Chanchamayo au Pérou, qui provoque dans le test d'incorporation de la thymidine tritiée dans les cellules de sarcome d'ascite de la souris selon Weitzel et coll. une inhibition de l'incorporation de la thymidine tritiée, qui est au moins la moitié de l'inhibition de l'incorporation par le Methotrexate®, sous forme physiologiquement acceptable pour application comme médicament.

2. Extrait selon la revendication 1, caractérisé en ce qu'au moins les tanins résorbables sont si réduits que d'une part leur activité est physiologiquement acceptable, mais que d'autre part ils peuvent toujours remplir une éventuelle fonction de substance support et/ou protectrice.

3. Extrait selon la revendication 1, que l'on peut obtenir en alcalinisant, pour enlever dans une large mesure les tanins, les parties de racines d'Uncaria tomentosa (WILLD.) DC. puis en extrayant avec un solvant organique, par exemple l'acétate d'éthyle.

4. Extrait selon la revendication 1, que l'on peut obtenir en extrayant à l'alcool les parties de racines d'Uncaria tomentosa (WILLD.) DC. pour retirer dans une large mesure les tanins, après quoi on mélange l'extrait avec une solution d'acétate de plomb et on retire le précipité apparu.

5. Extrait selon la revendication 1, que l'on peut obtenir en extrayant à l'eau les parties d'Uncaria tomentosa (WILLD.) DC. et en soumettant l'extrait à une transformation par fermentation pour retirer dans une large mesure les tanins à l'aide d'un schizomycète, après quoi on retire le précipité apparu.

6. Extrait selon la revendication 1, que l'on peut obtenir en soumettant à une transformation par fermentation les parties de racines d'une Uncaria tomentosa (WILLD.) DC. pour retirer dans une large mesure les tanins à l'aide d'un schizomycète puis en ce qu'on extrait en phase liquide, après quoi on met sous une forme physiologiquement acceptable l'extrait contenant la substance active.

7. Extrait selon les revendications 5 et 6, que l'on peut obtenir en effectuant la transformation par fermentation en milieu acide, de préférence à un pH compris entre 2 et 5.

8. Extrait selon la revendication 7, que l'on peut obtenir en effectuant la transformation par fermentation en présence d'acide citrique dilué.

9. Extrait selon l'une des revendications 5–8, que l'on peut obtenir en effectuant la transformation par fermentation avec un représentant de l'ordre des Trichodermes du groupe des Ascomycètes imparfaits.

10. Extrait selon la revendication 1, que l'on peut obtenirt en séchant des parties de racines d'une Uncaria tomentosa (WILLD.) DC. pour retirer dans une large mesure les tanins à une température qui se situe juste en-dessous de la température de carbonisation du bois, puis en extrayant en phase liquide, après quoi on met l'extrait contenant la substance active sous une forme physiologiquement acceptable.

11. Extrait selon la revendication 10, que l'on peut obtenir en effectuant le séchage à 130–150°C pendant un intervalle d'environ 45 min.

12. Extrait selon l'une des revendications 1 à 10, que l'on peut obtenir, après l'avoir débarrassé dans une large mesure des tanins, en concentrant, de préférence en séchant, et en dissolvant à nouveau de préférence dans le solvant d'extraction.

13. Extrait selon l'une des revendications 1 à 12, caractérisé en ce qu'on le lyophilise et en ce qu'on dissout la substance sèche dans un liquide physiologique, par exemple l'eau.

## Revendications pour l'Etat contractant: Autriche

1. Procédé de préparation d'une nouvelle substance pharmaceutiquement active, qui provoque dans le test d'incorporation de la thymidine tritiée dans les cellules de sarcome d'ascite de la souris selon Weitzel et coll. une inhibition de l'incorporation de la thymidine tritiée, qui est au moins la moitié de l'inhibition de l'incorporation par le Methotrexate®, caractérisé en ce que des parties de racines d'Uncaria tomentosa (WILLD.) DC. de l'ordre des gentianacées, avec coloration jaune-brun ou rouge foncé de la fibre fraîche, que l'on trouve par exemple dans le district de Chanchamayo au Pérou, sont extraites en phase liquide et débarrassées dans une large mesure des tanins, après quoi on met l'extrait sous une forme physiologiquement acceptable.

2. Procédé selon la revendication 1, caractérisé en ce qu'au moins les tanins résorbables sont si réduits que d'une part leur activité est physiologiquement acceptable, mais que d'autre part ils peuvent toujours remplir une éventuelle fonction de substance support et/ou protectrice.

3. Procédé selon la revendication 1, caractérisé en alcalinisant, pour enlever dans une large mesure les tanins, les parties de racines d'Uncaria tomentosa (WILLD.) DC. puis en extrayant avec un solvant organique, par exemple l'acétate d'éthyle.

4. Procédé selon la revendication 1, caractérisé en extrayant à l'alcool les parties de racines d'Uncaria tomentosa (WILLD.) DC. pour retirer dans une large mesure les tanins, après quoi on mélange l'extrait avec une solution d'acétate de plomb et on retire le précipité apparu.

5. Procédé selon la revendication 1, caractérisé en extrayant à l'eau les parties d'Uncaria tomentosa (WILLD.) DC. et en soumettant l'extrait à une transformation par fermentation pour retirer dans une large mesure les tanins à l'aide d'un schizomycète, après quoi on retire le précipité apparu.

6. Procédé selon la revendication 1, caractérisé en soumettant à une transformation par fermentation les parties de racines d'une Uncaria tomentosa (WILLD.) DC. pour retirer dans une large mesure les tanins à l'aide d'une schizomycète puis en ce qu'on extrait en phase liquide, après quoi on met sous une forme physiologiquement acceptable l'extrait contenant la substance active.

7. Procédé selon les revendications 5 et 6, caractérisé en effectuant la transformation par fermentation en milieu acide, de préférence à un pH compris entre 2 et 5.

8. Procédé selon la revendication 7, caractérisé en effectuant la transformation par fermentation en présence d'acide citrique dilué.

9. Procédé selon l'une des revendications 5–8, caractérisé en effectuant la transformation par fermentation avec un représentant de l'ordre des Trichodermes du groupe des Ascomycètes imparfaits.

10. Procédé selon la revendication 1, caractérisé en séchant des parties de racines d'une Uncaria tomentosa (WILLD.) DC. pour retirer dans une large mesure les tanins à une température qui se situe juste en-dessous de la température de carbonisation du bois, puis en extrayant en phase liquide, après quoi on met l'extrait contenant la substance active sous une forme physiologiquement acceptable.

11. Procédé selon la revendication 10, caractérisé en effectuant le séchage à 130–150°C pendant un intervalle d'environ 45 min.

12. Procédé selon l'une des revendications 1 à 10, caractérisé en ce que l'extrait est, après l'avoir débarrassé dans une large mesure des tanins, concentré, de préférence séché et dissolu à nouveau de préférence dans le solvant d'extraction.

13. Procédé selon l'une des revendications 1 à 12, caractérisé en ce qu'on le lyophilise et en ce qu'on dissout la substance sèche dans un liquide physiologique, par exemple l'eau.